# EUROPEAN PATENT SPECIFICATION

(11) **EP 0 451 199 B1**
(45) Date of publication and mention of the grant of the patent: **21.09.1994**
(21) Application number: 90901439.1
(22) Date of filing: 21.12.1989
(51) Int. Cl.: C07C 19/08, C07C 17/00

(54) **PROCESSES USING ALUMINUM FLUORIDE CATALYST COMPOSITIONS FOR PREPARING HALOETHANES CONTAINING FLUORIDE**
VERFAHREN ZUR HERSTELLUNG VON FLUORENTHALTENDEN HALOETHANEN MIT VERWENDUNG VON ALUMINIUMFLUORID ENTHALTENDEN KATALYSATORZUSAMMENSTELLUNGEN
PROCEDES UTILISANT DES COMPOSITIONS DE FLUORURE D'ALUMINIUM DE CATALYSE POUR LA PREPARATION D'HALOETHANES CONTENANT DU FLUOR

(30) Priority: 28.12.1988 US 291100
(43) Date of publication of application: 16.10.1991
(73) Proprietor: E.I. DU PONT DE NEMOURS AND COMPANY, Wilmington Delaware 19898 (US)
(72) Inventor: MANZER, Leo, Ernest, Wilmington, DE 19803 (US); TEBBE, Frederick, Nye, Hockessin, DE 19707 (US)
(74) Representative: Jones, Alan John
(86) International application number: PCT/US89/05732
(87) International publication number: WO 90/07481

(56) References cited:
- EP-A- 317 981
- US-A- 2 748 177
- US-A- 3 650 987
- Gervasutti, C. et al., J. Fluor. Chem., vol 19(1981/82), pp. 1-20

## Description

This invention relates to catalysts and their use for the manufacture of haloethanes such as 1,1-dichloro-1,2,2,2-tetrafluoroethane (i.e. "CFC-114a"), and more particularly to the use of aluminum fluoride catalysts and use thereof for preparing haloethanes containing fluoride such as CFC-114a.

### BACKGROUND OF THE INVENTION

Fluorinated aluminas are well known as fluorination or chlorofluorination catalysts. For example the use of both aluminum fluoride and aluminum fluoride containing iron, chromium and nickel for fluorination or chlorofluorination reactions is described in U.S. Patent No. 3,650,987. The fluorided alumina is often prepared by the addition of HF to Al₂O₃. The products from these reactions contain large amounts of the symmetrical isomers of various chlorofluorocarbons. The addition of the metal dopants increases the amount of symmetrical isomers.

German (DDR) Patent Specification 117,580 discloses a process for the preparation of asymmetrical fluorochlorocarbon compounds of the C₂ series (e.g. CFC-114a) by the reaction of tetrachloroethylene, chlorine and hydrogen fluoride over a metal doped aluminum fluoride catalyst. In the example of this patent with the highest amount of asymmetrical products, the 1,1-dichloro-1,2,2,2-tetrafluoroethane (CFC-114a)/1,2-dichloro-1,1,2,2-tetra-fluoroethane (CFC-114) ratio is about 11.5; and the 1,1,1-trichloro-2,2,2-trifluoroethane (CFC-113a)/1,1,2-trichloro-1,2,2-trifluoroethane (CFC-113) ratio is about 8.0. However 57.4% of the product is chloropentafluoroethane (CFC-115), which is generally considered an undesirable byproduct in CFC-114a manufacturing processes. In another example where 6.1% of the product is CFC-115, the ratios of CFC-114a/CFC-114 and CFC-113a/CFC-113 are 7.3 and 3.8 respectively.

European Patent Application 317,981 discloses a process for isomerizing CFC-113 to CFC-113a followed by fluorination with HF to produce CFC-114a. As isomerization catalyst, a halide or oxide of Al, Cr, Mg, Ca, Sr, Ba, Fe, Ni, or Co may be used. In the example of this patent with the highest ratio of CFC-114a/CFC-114 (52.7) the ratio of CFC-113a/CFC-113 is 1.1. In other examples the CFC-114a/CFC-114 ratio varied from 45.3 to 5.5 and the CFC-113a/CFC-113 ratio from 6.2 to 1.1.

Japanese Kokai 1-172347 discloses a process for the preparation of CFC-114a by first disproportionating CFC-114 to CFC-113a followed by reaction with HF. The disproportionation catalyst is a halide or oxide of Al, Cr, Mg, Ca, Sr, Ba, Fe, Ni, or Co. The fluorination catalyst is either a halide or an oxide of Al, Cr, Mg, Ca, Sr, Ba, Fe, Ni, Co, Sb, Nb or Ta. In the examples the CFC-114a/CFC-114 ratios vary from 9.0 to 16.7 and the CFC-113a/CFC-113 ratios from 0.1 to >36. In the high 113a/113 ratio example the yield of 114a is only 15.0%. In the other examples the yields of 114a varied from 43.0% to 51.0%.

It is also known in the art (GB 1,578,933) that both CClF₂CClF₂ (CFC-114) and CF₃CCl₂F (CFC-114a) may be hydrogenated over a Pd catalyst to CHF₂CHF₂ (HFC-134) and CF₃CH₂F (HFC-134a) respectively. The latter compound (HFC-134a) is considered a refrigerant for replacing CCl₂F₂ since it does not significantly contribute to stratospheric ozone depletion while CCl₂F₂ is suspected of being a major contributor. Other haloethanes containing fluoride are considered useful as refrigerants, blowing agents, solvents and/or as reagents for preparing such products.

Accordingly, there is continued interest in developing economic and efficient processes for preparing haloethanes containing fluoride such as 1,1-dichloro-1,2,2,2-tetrafluoroethane.

### SUMMARY OF THE INVENTION

A process disclosed herein involves transforming a reactant having the formula C₂HₓCl_{y}F_{z} wherein x is zero or an integer up to 3, y is zero or an integer up to 6, z is zero or an integer up to 4, and the sum of x, y and z is 6, into a product which is different from said reactant and has the formula C₂HₐCl_{b}F_{d} where a is zero or an integer up to 3, b is zero or an integer up to 5, d is an integer from 1 to 4, and the sum of a, b and d is 6, and where d is at least z, and provided that when $\text{y = b = 0}$ , a cannot be equal to x. The process involves using aluminum fluoride catalyst wherein a gaseous mixture comprising the reactant and HF is contacted with a catalyst composition at an elevated temperature, wherein said catalyst composition consists essentially of an aluminum fluoride prepared by the reaction of aluminum hydroxide and HF. In accordance with the process of this invention, at least one compound selected from CCl₃CCl₃, CCl₃CCl₂F, CCl₂FCCl₂F, CClF₂CCl₃, and CCl₂FCClF₂ may be transformed into 1,1-dichloro-1,2,2,2-tetrafluoroethane. The 1,1-dichloro-1,2,2,2-tetrafluoroethane prepared by the process may be hydrodechlorinated to produce 2-chloro-1,1,1,2-tetrafluoroethane and/or tetrafluoroethane.

### DETAILED DESCRIPTION OF THE INVENTION

A saturated reactant having the formula C₂HₓCl_{y}F_{z} wherein x is zero or an integer up to 3, y is zero or an integer up to 6, z is zero or an integer up to 4, and the sum of x, y and z is 6, may be transformed into a saturated product which is different from said reactant and has the formula C₂HₐCl_{b}F_{d} where a is zero or an integer up to 3, b is zero or an integer up to 5, d is an integer from 1 to 4, and the sum of a, b and d is 6, and where d is at least z, and provided that when $\text{y = b = 0}$ , a cannot be equal to x, by using the specified aluminum fluoride catalyst. The transformations involved in these processes can include various types of conversion reactions such as fluorination, chlorofluorination, isomerization, disproportionation, and combinations of such reactions. One technique for transforming a reactant to a product comprises the step of contacting a gaseous mixture comprising HF with a catalyst composition at an elevated temperature, wherein said catalyst composition consists essentially of an aluminum fluoride prepared by the reaction of aluminum hydroxide and HF. Indeed a process for transforming at least one compound selected from CCl₃CCl₃, CCl₃CCl₂F, CCl₂FCCl₂F, CClF₂CCl₃, and CCl₂FCClF₂ into 1,1-dichloro-1,2,2,2-tetrafluoroethane by contacting a gaseous mixture comprising HF and said at least one compound with an aluminum fluoride catalyst at an elevated temperature is provided in accordance with this invention, and is characterized by using a catalyst composition consisting essentially of an aluminum fluoride prepared by the reaction of aluminum hydroxide and HF. The most preferred reactant for this process is 1,1,2-trichloro-1,2,2-trifluoroethane.

The interaction of the reactant with HF and optionally Cl₂ in the presence of the catalyst composition of the instant invention is conducted at an elevated temperature. Suitable temperatures are generally within the range of about 200°C to 475°C. Preferably the temperature is within the range of about 300°C to 400°C, and most preferably is within the range of about 350°C to 375°C. Contact times can influence the yield of the reaction to some extent. Preferably the temperature and contact time are balanced to achieve a desirable yield. For example, in the process of this invention where CFC-113 is transformed into CFC-114a, the temperature and contact time are preferably controlled to achieve the desired product yield (e.g. at least about 95% total of CFC-114a and recyclable by-products). Preferably the aluminum fluoride purity of the catalyst composition is sufficient to provide a molar ratio of 1,1-dichloro-1,2,2,2-tetrafluoroethane to 1,2-dichloro-1,1,2,2-tetrafluoroethane (i.e. CFC-114a to CFC-114) of at least about 45 and most preferably sufficient to provide a molar ratio of CFC-114a to CFC-114 of at least about 47.5. A contact time within the range of about 5 to 100 seconds is typical. Preferably the contact time is within the range of about 10 to 90 seconds, and most preferably is within the range of about 15 to 60 seconds.

The HF in the gaseous mixture is preferably at least the stoichiometric amount needed to produce the product (e.g. CFC-114a). The molar ratio of the HF to the reactant is typically within the range of about 1:1 to 20:1. In the process of this invention where CFC-113 is transformed into CFC-114a, the ratio of HF to CFC-113 is preferably within the range of about 1:1 to 10:1, and is most preferably within the range of about 1:1 to 3:1.

Typically Cl₂ is not needed to produce the product. However it can nevertheless be optionally added to facilitate the reaction. For example, in the process of this invention where CFC-113 is transformed into CFC-114a, the gaseous mixture may optionally comprise Cl₂; and the molar ratio of Cl₂ when present, to the reactant (CFC-113) is preferably within the range of about 0.01:1 to 2:1, and most preferably is about 1:1.

Products such as CFC-114a can be separated by a usual method such as fractional distillation. By-products having no more fluorine atoms per molecule than the desired product are considered recyclable, and may advantageously be further contacted with the catalyst composition used in this invention. Thus, for CFC-114a production, by-products such as C₂Cl₅F, C₂Cl₄F₂, C₂F₃Cl₃ and CClF₂CClF₂ are considered recyclable.

The reaction of the reactant with HF (and, optionally chlorine) may be conducted in any suitable reactor, including fixed and fluidized bed reactors which are charged with the catalyst compositions. The reaction vessel should be constructed from materials which are resistant to the corrosive effects of HF and Cl₂ such as Hastelloy® nickel alloy and Inconel® nickel alloy. Optionally, before the catalyst is contacted by the reactant, it may be pretreated with gaseous HF.

Pressure is not critical. Atmospheric and superatmospheric pressures are the most convenient and are therefore preferred.

1,1-dichloro-1,2,2,2-tetrafluoroethane produced by this invention has utility as a solvent and as an intermediate for the preparation of 2-chloro-1,1,1,2-tetrafluoroethane and/or 1,1,1,2-tetrafluoroethane. Indeed an improved process for producing 1,1,1,2-tetrafluoroethane and/or 2-chloro-1,1,2,2-tetrafluoroethane by hydrodechlorinating 1,1-dichloro-1,2,2,2-tetrafluoroethane is provided in accordance with this invention. The improvement comprises the step of preparing CFC-114a by using the reactant 1,1,2-trichloro-1,2,2-trifluoroethane and the catalyst compositions of this invention as described above. Hydrodechlorination is described in Journal of Fluorine Chemistry 19(1981/82)1-20 and involves reacting 1,1,-dichloro-1,2,2,2-tetrafluoroethane with hydrogen at 120° to 240°C for 10 to 35 seconds with a molar ratio of hydrogen to the dichlorotetrafluoroethane of 2 to 4 in the presence of palladium on charcoal catalyst.

The catalyst compositions used in accordance with this invention consist essentially of an aluminum fluoride prepared by the reaction of aluminum hydroxide with HF.

The catalyst composition of this invention may be suitably prepared by reacting aqueous HF (e.g. 48% solution) with aluminum hydroxide. Suitable aluminum hydroxide may be prepared by the hydrolysis of AlR₃, where each R is independently selected from C₁ to C₆ alkyl groups. For example, a preparation of high purity Al(OH)₃ prepared by the hydrolysis of Al(CH₂CH₃)₃ is described by F. N. Tebbe et al., J. Am. Ceram. Soc., 71 [4], C-204 - C-206, (1988). Suitable aluminum hydroxide may also be prepared by hydrolysis of Al(OR)₃, where each R is independently selected from C₁ to C₆ alkyl groups. For example, high purity Al(OH)₃ may be prepared by the hydrolysis of Al(OCH(CH₃)CH₂CH₃)₃.

A particularly useful catalyst composition consisting essentially of an aluminum fluoride is prepared by dissolving aluminum hydroxide in aqueous HF; evaporating the resulting solution to obtain a residue of an aluminum fluoride; and heating said residue to produce a dried solid. The processes of this invention include the chlorofluorination of CF₃CCl₃, CF₃CHClF and/or CF₃CH₃ to CFC-114a, and fluorination of CF₃-CHCl₂ to CF₃-CHClF.

Practice of the invention will become further apparent from the following non-limiting Examples. In these Examples inches may be converted to cm by multiplying by 2.54 and feet may be converted into cm by multiplying by 30.48.

### EXAMPLE I

Aluminum sec-butoxide (Alfa, 95%), about 300 g, was placed in an open dish and allowed to hydrolyze in air over two days. The aluminum hydroxide produced was a powder and clusters of powdery solids. In a polyethylene tray a portion of the aluminum hydroxide (39 g) was dissolved in 48% aqueous hydrofluoric acid (100 mL). The volatiles were evaporated at ambient temperature over three days in a fume hood. The resulting white residue was dried at 110°C for 48 hours. The solid was then heated in air at a rate of 5°C/min to 500°C, and held at this temperature for three hours. After cooling, the solid (30.3 g) was crushed and passed through a sieve to yield fines (12.8 g) and a fraction of granules 12x20 mesh in size (17.5 g). Analysis showed 31.5% Al and 227 ppm (0.0227%) of metal ion impurities.

### EXAMPLE II

A reactor (a 0.5 inch ID, 12 inch long Inconel® nickel alloy pipe) was charged with aluminum fluoride (15.7 g, 25 mL) prepared as described in Example I, and placed in a sand bath. The bath was gradually heated to 250°C while nitrogen gas at a flow rate of 50cc/minute was passed through the reactor to remove traces of water. HF and nitrogen gas (1:4 molar ratio) were then passed through the reactor. An exotherm of about 10°C, which travelled down the reactor, was observed. The temperature was gradually raised to 450°C, the nitrogen flow decreased with time until neat HF was passed through the reactor. The HF flow was stopped after no further exotherm was recorded. HF/CCl₂FCClF₂/Cl₂ in a 5/1/1 molar ratio was then passed over the catalyst at 375°C and a 15 second contact time. The reactor effluent was sampled on-line with a Hewlett-Packard 5890 gas chromatograph using a 20 foot long, 1/8" diameter, column containing Krytox® perfluorinated polyether on an inert support and a helium flow of 35 cc/minute. Gas chromatographic conditions were 70°C for 3 minutes followed by temperature programming to 180°C at a rate of 6°C/minute. Analysis showed the following to be present (area percent): 0.1% CCl₂FCCl₂F/CClF₂CCl₃, 3.8% CCl₂FCClF₂ (CFC-113), 10.7% CF₃CCl₃ (CFC-113a), 1.8% CClF₂CClF₂ (CFC-114), 83.3% CF₃CCl₂F (CFC-114a), and 0.5% CClF₂CF₃ (CFC-115).

The yield to useful products is greater than 99% and the molar ratio of CFC-114a/CFC-114 is 46.

### EXAMPLE III

After the run of Example II above, HF/CCl₂FCClF₂ in a 5/1 molar ratio was then passed through the reactor and over the catalyst at 400°C and a 17 second contact time. The reactor effluent was sampled on-line with a Hewlett-Packard 5890 gas chromatograph using a 20 foot long, 1/8" diameter, column containing Krytox® perfluorinated polyether on an inert support and a helium flow of 35 cc/minute. Gas chromatographic conditions were 70°C for 3 minutes followed by temperature programming to 180°C at a rate of 6°C/minute. Analysis showed the following to be present (area percent): 0.1% CCl₂FCClF₂ (CFC-113), 1.4% CF₃CCl₃ (CFC-113a), 5.9% CClF₂CClF₂ (CFC-114), 89.6% CF₃CCl₂F (CFC-114a), and 3.1% CClF₂CF₃ (CFC-115).

The yield to useful products is greater than 96% and the molar ratio of CFC-114a/CFC-114 is 15.

## Claims

1. A process for transforming a reactant having the formula C₂HₓCl_{y}F_{z} wherein x is zero or an integer up to 3, y is zero or an integer up to 6, z is zero or an integer up to 4, and the sum of x, y and z is 6, into a product which is different from said reactant and has the formula C₂HₐCl_{b}F_{d} where a is zero or an integer up to 3, b is zero or an integer up to 5, d is an integer up to 4, and the sum of a, b and d is 6, and where d is at least z and provided that when $\text{y=b=0}$ , a cannot be equal to x, wherein a gaseous mixture comprising the reactant and HF is contacted with a catalyst composition at an elevated temperature, said catalyst composition consisting essentially of an aluminium fluoride prepared by the reaction of aluminium hydroxide and HF.

2. The process of claim 1 wherein at least one reactant selected from CCl₃CCl₃, CCl₃CCl₂F, CCl₂FCCl₂F, CClF₂CCl₃_{,} and CCl₂FCClF₂ is transformed into 1,1-dichloro-1,2,2,2-tetrafluoroethane.

3. The process of claim 2 wherein by-products having less than five fluorine atoms per molecule are recycled and the gaseous mixture further comprises Cl₂.

4. The process of either of claims 1 and 2 wherein the molar ratio of said HF to said reactant is within the range of about 1:1 to 20:1.

5. The process of claim 3 wherein the molar ratio of said Cl₂ to said reactant is within the range of about 0.01:1 to 2:1.

6. The process of any one of the preceding claims wherein before the catalyst is contacted by said reactant it is pretreated with HF gas.

7. The process of any one of the preceding claims wherein said aluminium hydroxide is prepared by the hydrolysis of AlR₃ or the hydrolysis of Al(OR)₃ where each R is independently selected from C₁ to C₆ alkyl groups and the elevated temperature is within the range of about 200°C to 475°C.

8. The process of any one of claims 2 to 7 wherein said reactant is 1,1,2-trichloro-1,2,2-trifluoroethane.

9. The process of claim 1 wherein the aluminium fluoride purity of the catalyst composition is sufficient to provide a molar ratio of 1,1-dichloro-1,2,2,2-tetrafluoroethane to 1,2-dichloro-1,1,2,2-tetrafluoroethane of about 45.

10. A process for producing 2-chloro-1,1,1,2-tetrafluoroethane and/or 1,1,1,2-tetrafluoroethane by hydrodechlorinating 1,1-dichloro-1,2,2,2-tetrafluoroethane, wherein in a first step 1,1-dichloro-1,2,2,2-tetrafluoroethane is prepared according to the process of claim 1 by contacting a gaseous mixture comprising 1,1,2-trichloro-1,2,2-trifluoroethane and HF at a temperature within the range of 200°C to 475°C in the presence of a catalyst composition consisting essentially of an aluminium fluoride prepared by the reaction of aluminium hydroxide and hydrofluoric acid and in a second step 1,1-dichloro-1,2,2,2-tetrafluoroethane is hydrodechlorinated to produce said 2-chloro-1,1,1,2-tetrafluoroethane and/or 1,1,1,2-tetrafluoroethane.

## Patentansprüche

1. Verfahren zum Umwandeln eines Reaktanten der Formel C₂HₓCl_{y}F_{z}, worin x 0 oder eine ganze Zahl bis 3 ist, y 0 oder eine ganze Zahl bis 6 ist, z 0 oder eine ganze Zahl bis 4 ist und die Summe von x, y und z 6 ist, in ein Produkt, das von dem Reaktanten verschieden ist und die Formel C₂HₐCl_{b}F_{d} hat, worin a 0 oder eine ganze Zahl bis 3 ist, b 0 oder eine ganze Zahl bis 5 ist, d eine ganze Zahl bis 4 ist und die Summe von a, b und d 6 ist, und wo d wenigstens z ist, und mit der Maßgabe, daß wenn $\text{y = b = 0}$ , a nicht gleich x sein kann, worin eine gasförmige, den Reaktanten und HF umfassende Mischung mit einer Katalysator-Zusammensetzung bei erhöhter Temperatur in Berührung gebracht wird, wobei die Katalysator-Zusammensetzung im wesentlichen aus einem Aluminiumfluorid besteht, das durch die Reaktion von Aluminiumhydroxid und HF hergestellt ist.

2. Verfahren nach Anspruch 1, worin wenigstens ein Reaktant, der aus CCl₃CCl₃, CCl₃CCl₂F, CCl₂FCCl₂F, CClF₂CCl₃ und CCl₂FCClF₂ ausgewählt ist, in 1,1-Dichlor-1,2,2,2-tetrafluorethan umgewandelt wird.

3. Verfahren nach Anspruch 2, worin Nebenprodukte mit weniger als fünf Fluor-Atomen pro Molekül im Kreislauf zurückgeführt werden und die gasförmige Mischung weiterhin Cl₂ umfaßt.

4. Verfahren nach einem der Ansprüche 1 und 2, worin das Stoffmengen-Verhältnis des HF zu dem Reaktanten innerhalb des Bereichs von etwa 1 : 1 bis 20 : 1 liegt.

5. Verfahren nach Anspruch 3, worin das Stoffmengen-Verhältnis des Cl₂ zu dem Reaktanten innerhalb des Bereichs von etwa 0,01 : 1 bis 2 : 1 liegt.

6. Verfahren nach irgendeinem der vorhergehenden Ansprüche, worin der Katalysator, bevor er mit dem Reaktanten in Berührung gebracht wird, mit HF-Gas vorbehandelt wird.

7. Verfahren nach irgendeinem der vorhergehenden Ansprüche, worin das Aluminiumhydroxid durch die Hydrolyse von AlR₃ oder die Hydrolyse von Al(OR)₃ hergestellt wird, worin R jeweils unabhängig aus C₁- bis C₆-Alkyl-Gruppen ausgewählt ist und die erhöhte Temperatur innerhalb des Bereichs von etwa 200 °C bis 475°C liegt.

8. Verfahren nach irgendeinem der Ansprüche 2 bis 7, worin der Reaktant 1,1,2-Trichlor-1,2,2-trifluorethan ist.

9. Verfahren nach Anspruch 1, worin die Aluminiumfluorid-Reinheit der Katalysator-Zusammensetzung ausreicht, um ein Stoffmengen-Verhältnis des 1,1-Dichlor-1,2,2,2-tetrafluorethans zu 1,2-Dichlor-1,1,2,2-tetrafluorethan von etwa 45 bereitzustellen.

10. Verfahren zur Herstellung von 2-Chlor-1,1,1,2-tetrafluorethan und/oder 1,1,1,2-Tetrafluorethan durch Hydrodechlorieren von 1,1-Dichlor-1,2,2,2-tetrafluorethan, worin in einem ersten Schritt 1,1-Dichlor-1,2,2,2-tetrafluorethan nach dem Verfahren des Anspruchs 1 durch In-Berührung-Bringen einer 1,1,2-Trichlor-1,2,2-trifluorethan und HF umfassenden gasförmigen Mischung bei einer Temperatur im Bereich von 200 °C bis 475 °C in Gegenwart einer Katalysator-Zusammensetzung, die im wesentlichen aus einem Aluminiumfluorid besteht, das durch die Reaktion von Aluminiumhydroxid und HF hergestellt ist, und in einem zweiten Schritt 1,1-Dichlor-1,2,2,2-tetrafluorethan hydrodechloriert wird, um das genannte 2-Chlor-1,1,1,2-tetrafluorethan und/oder 1,1,1,2-Tetrafluorethan zu erzeugen.

## Revendications

1. Procédé pour transformer un réactif ayant la formule C₂HₓCl_{y}F_{z} où x est zéro ou un nombre entier jusqu'à 3, y est zéro ou un nombre entier jusqu'à 6, z est zéro ou un nombre entier jusqu'à 4, et la somme de x, y et z est 6, en un produit qui est différent dudit réactif et qui a la formule C₂HₐCl_{b}F_{d} où a est zéro ou un nombre entier jusqu'à 3, b est zéro ou un nombre entier jusqu'à 5, d est un nombre entier jusqu'à 4, et la somme de a, b et d est 6, et où d est au moins z et à condition que lorsque $\text{y=b=0}$ , a ne peut pas être égal à x, dans lequel un mélange gazeux comprenant le réactif et HF est mis en contact avec une composition de catalyseur à une température élevée, ladite composition de catalyseur étant essentiellement constituée d'un fluorure d'aluminium préparé par la réaction d'hydroxyde d'aluminium et de HF.

2. Procédé selon la revendication 1, dans lequel au moins un réactif choisi parmi CCl₃CCl₃, CCl₃CCl₂F, CCl₂FCCl₂F, CClF₂CCl₃, et CCl₂FCClF₂ est transformé en 1,1-dichloro-1,2,2,2-tétrafluoroéthane.

3. Procédé selon la revendication 2, dans lequel les sous-produits ayant moins de cinq atomes de fluor par molécule sont recyclés et le mélange gazeux comprend en outre Cl₂.

4. Procédé selon l'une quelconque des revendications 1 et 2, dans lequel le rapport molaire dudit HF audit réactif est dans la gamme d'environ 1:1 à 20:1.

5. Procédé selon la revendication 3, dans lequel le rapport molaire dudit Cl₂ audit réactif est dans la gamme d'environ 0,01:1 à 2:1.

6. Procédé selon l'une quelconque des revendications précédentes, dans lequel avant que le catalyseur ne soit contacté par ledit réactif, il est prétraité avec du gaz HF.

7. Procédé selon l'une quelconque des revendications précédentes, dans lequel ledit hydroxyde d'aluminium est préparé par l'hydrolyse de AlR₃ ou l'hydrolyse de Al(OR)₃ où chaque R est indépendamment choisi parmi les groupes alkyles en C₁ à C₆ et la température élevée est dans la gamme d'environ 200 °C à 475 °C.

8. Procédé selon l'une quelconque des revendications 2 à 7, dans lequel ledit réactif est le 1,1,2-trichloro-1,2,2-trifluoroéthane.

9. Procédé selon la revendication 1, dans lequel la pureté du fluorure d'aluminium de la composition de catalyseur est suffisante pour fournir un rapport molaire du 1,1-dichloro-1,2,2,2-tétrafluoroéthane au 1,2-dichloro-1,1,2,2-tétrafluoroéthane d'environ 45.

10. Procédé pour préparer du 2-chloro-1,1,1,2-tétrafluoroéthane et/ou du 1,1,1,2-tétrafluoroéthane par hydrodéchloration de 1,1-dichloro-1,2,2,2-tétra-fluoroéthane, dans lequel, dans une première étape, du 1,1-dichloro-1,2,2,2-tétrafluoroéthane est préparé selon le procédé de la revendication 1 en mettant en contact un mélange gazeux comprenant du 1,1,2-trichloro-1,2,2-trifluoroéthane et HF à une température dans la gamme de 200 °C à 475 °C en présence d'une composition de catalyseur essentiellement constituée d'un fluorure d'aluminium préparé par la réaction d'hydroxyde d'aluminium et d'acide fluorhydrique et, dans une seconde étape, du 1,1-dichloro-1,2,2,2-tétrafluoroéthane est hydrodéchloré pour produire lesdits 2-chloro-1,1,1,2-tétrafluoroéthane et/ou 1,1,1,2-tétrafluoroéthane.
